# EUROPÄISCHE PATENTSCHRIFT

(11) **EP 0 581 099 B1**
(45) Veröffentlichungstag und Bekanntmachung des Hinweises auf die Patenterteilung: **22.01.1997**
(21) Anmeldenummer: 93111122.3
(22) Anmeldetag: 12.07.1993
(51) Int. Cl.: C07C 209/28

(54) **Verfahren zur Herstellung von Diphenylaminen**
Process for the preparation of diphenylamines
Procédé pour la préparation de diphénylamines

(30) Priorität: 23.07.1992 DE 4224366
(43) Veröffentlichungstag der Anmeldung: 02.02.1994
(73) Patentinhaber: BAYER AG, 51368 Leverkusen (DE)
(72) Erfinder: Immel, Otto,Dr., D-47803 Krefeld (DE); Darsow, Gerhard, Dr., D-47804 Krefeld (DE); Buysch, Hans-Josef, Dr., D-47809 Krefeld (DE)

(56) Entgegenhaltungen:
- EP-A- 0 103 990
- DE-A- 1 493 942
- DE-A- 3 801 754

## Beschreibung

Die Erfindung betrifft ein Verfahren zur Herstellung von gegebenenfalls substituierten Diphenylaminen durch Umsetzung der zugrundeliegenden Cyclohexanone und Aniline in Gegenwart eines Trägerkatalysators, der eines oder mehrere dehydrierend wirkende(s) Metall(e) der unten beschriebenen Art enthält.

Zur Herstellung von Diphenylamin und dessen Derivaten ist aus der DE-OS 2 331 878 ein Verfahren bekannt, bei dem man von Iminen, wie N-Cyclohexyliden-anilin und dessen Derivaten, ausgeht und sie in der Gasphase in Gegenwart von Trägerkatalysatoren auf der Basis von Nickel, Platin, Palladium oder Kupfer-Chrom dehydriert.

Weiterhin ist aus DE-OS 2 520 893 bekannt, Diphenylamin durch katalytische Dehydrierung von Verbindungen und/oder Verbindungsgemischen, die aus ganz oder teilweise hydriertem Diphenylamin bestehen, in Gegenwart eines Nickel-Chrom, Aluminium, Kupfer, Mangan und Alkali enthaltenden Dehydrierungskatalysators herzustellen. Als solche Verbindungen werden zweikernige aromatische Imine in den Ausführungsbeispielen gezeigt.

Gemäß DE-OS 14 93 942 erfolgt die Herstellung von Diphenylamin aus Cyclohexanon und Anilin in der flüssigen Phase über die Zwischenstufe der zugehörigen Schiffschen Base in Gegenwart eines Dehydrierungskatalysators und eines Wasserstoffakzeptors. Ein solches Verfahren ist für die technische Realisierung zu teuer.

Auch die Dehydrierung von Dicyclohexylamin zu Diphenylamin an Edelmetall-Katalysatoren wurde bereits vorgeschlagen (DE-OS 3 801 754).

Die genannten Verfahren erfordern relativ kostspielig herzustellende Ausgangsmaterialien, wie N-Cyclohexyliden-anilin oder Dicyclohexylamin und sind für ein industriell durchzuführendes Verfahren verbesserungsbedürftig.

In der DE-OS 22 49 089 wird ein Verfahren zur Herstellung von Diphenylamin vorgeschlagen, wonach man Anilin in Dampfform über einen Aluminiumoxidkatalysator leitet, der mit Bortrifluorid behandelt wird oder worden ist. Die zu erzielenden Raum-Zeit-Ausbeuten sind aber für eine technische Produktion von Diphenylamin gering.

Gemäß der deutschen Patentschrift 530 736 werden Anilin und Phenol bei erhöhter Temperatur in Gegenwart von mit Säuren behandelten Katalysatoren (Kaolin oder Bleicherde) umgesetzt. Die Reaktion erfolgt mit einem Anilinüberschuß, wobei aber ein Teil des Anilins verloren geht.

Die Erfindung betrifft ein Verfahren zur Herstellung von Diphenylaminen der Formel 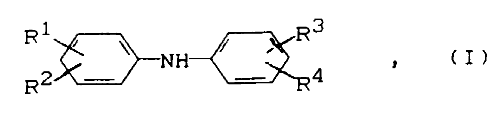 in der
- R¹, R², R³ und R⁴: unabhängig voneinander Wasserstoff C₁-C₄-Alkyl oder C₁-C₄-Alkoxy bedeuten,
das dadurch gekennzeichnet ist, daß Aniline der Formel 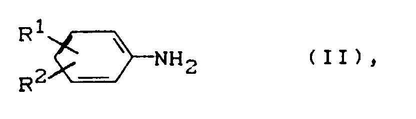 mit Cyclohexanonen der Formel 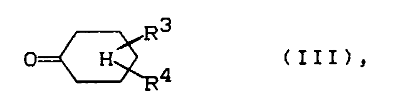 die sich im Gemisch mit recyclisierten Reaktionsprodukten befinden können, wobei in den Formeln die Reste R¹ - R⁴ die obige Bedeutung haben, und wobei Anilin : Cyclohexanon im molaren Verhältnis von 1:4-6:1, bevorzugt 1:2-5:1, vorliegen, an einem 0,05-5 Gew.-%, bevorzugt 0,05-4 Gew.-%, besonders bevorzugt 0,1-3 Gew.-%, bezogen auf das Gesamtgewicht des Katalysators, eines oder mehrere dehydrierend wirkende(s) Metall(e) enthaltenden Trägerkatalysator bei 200-450°C und 0,1-20 bar in der Gasphase umgesetzt werden, wobei das (die) Metall(e) der Gruppe aus Ru, Pd, Os, Ir, Pt, Fe, Co, Ni, Re, Mn, Cu, Ag, Cr, Ce angehören.

Im erfindungsgemäßen Verfahren vollzieht sich offenbar zunächst eine Kondensation von Anilin und Cyclohexanon, von der angenommen werden kann, daß sie unter Wasser-austritt zum N-Cyclohexyliden-anilin führt, welches dann zum Diphenylamin dehydriert wird. Der erfolgreiche Verlauf des erfindungsgemäßen Verfahrens ist in verschiedener Weise überraschend. So ist bekannt, daß die Bildung des N-Cyclohexyliden-anilins eine Gleichgewichtsreaktion darstellt, die stets nennenswerte Teile der Ausgangsprodukte zuläßt. So mußte man stets mit der Gefahr rechnen, daß das Cyclohexanon in erhöhtem Maße zu in diesem Verfahren wertlosem Phenol aromatisiert wird. Des weiteren wäre zu erwarten gewesen, daß die intermediäre Bildung des N-Cyclohexyliden-anilins nicht sehr begünstigt ist, da zu seiner Bildung ausgesprochen saure Katalysatoren erforderlich sind.

Das erfindungsgemäße Verfahren birgt insoweit ein weiteres Überraschungsmoment, als nicht nur, wie oben geschildert wurde, die weitgehende Bildung von Phenol aus dem Cyclohexanon unterbleibt, sondern sogar zugesetztes Phenol im Sinne von Cyclohexanon reagiert, und damit im Sinne des oben geschilderten Denkmodells sogar eine Umwandlung von Phenol in Cyclohexanon stattfinden muß. Die Menge dieses zugesetzten Phenols, das zur Vermeidung von unnötigen Produktgemischen das gleiche Substitutionsmuster wie das eingesetzte Cyclohexanon haben soll, beträgt 0-20 mol-%, bezogen auf die Gesamtmenge des Gemisches aus Cyclohexanon und Phenol.

Aber auch der Fall einer Nettoproduktion von Phenol, etwa bei höheren molaren Cyclohexanon-Anilin-Verhältnissen ist erfindungsgemäß zulässig, weil im Zuge einer destillativen Aufarbeitung des Reaktionsgemisches Phenol abgetrennt werden kann, Eine solche Abtrennung erfolgt vorzugsweise gemeinsam mit nicht umgesetztem Anilin. Daraus ergeben sich beispielsweise folgende Verwendungsmöglichkeiten für das Phenol: 1. kann bei geringen Mengen an Phenol das Phenol-Anilin-Gemisch zu reinem Anilin aufgearbeitet werden, wobei der kleine Phenolanteil beispielsweise verbrannt wird; 2. kann das Phenol-Anilin-Gemisch bei verändertem molaren Cyclohexanon-Anilin-Verhältnis in das erfindungsgemäße Verfahren zurückgeführt werden; 3. kann das Phenol-Anilin-Gemisch bei größerem Phenolanteil beispielsweise einer hydrierenden Umsetzung zu Dicyclohexylamin oder einem anderen geeigneten Zweck zugeführt werden. Phenol im Reaktionsgemisch des erfindungsgemäßen Verfahrens ist daher im Bereich von 0-25 Gew.-%, bevorzugt 0,1-20 Gew.-%, bezogen auf das gesamte Reaktionsgemisch, zulässig.

Der erfindungsgemäß einzusetzende Katalysator enthält eines oder mehrere Metall(e) aus der Gruppe von Ru, Pd, Os, Ir, Pt, Fe, Co, Ni, Re, Mn, Cu, Ag, Ce und Cr. Die Metalle liegen in einer Gesamtmenge von 0,05-5 Gew.-%, bevorzugt 0,05-4 Gew.-%, besonders bevorzugt 0,1-3 Gew.-% vor, bezogen auf das Gesamtgewicht des Katalysators. In bevorzugter Weise liegt eines oder mehrere Metall(e) aus der Gruppe von Pd, Ir, Pt, Co, Ni, Re, Mn, Ce und Cr vor.

In weiterhin bevorzugter Weise enthält der erfindungsgemäß einzusetzende Katalysator eine Kombination von Pt mit mindestens einem der genannten anderen Platinmetalle oder Re, in der Pt in einer Menge von 10 -90 % des Gesamtgewichts aller Metalle vorliegt. In besonders bevorzugter Weise wird Pt mit Pd, Ir oder Re oder einem Gemisch aus Pd, Ir und Re kombiniert. In ganz besonders bevorzugter Weise wird zur Kombination mit dem Pt das Pd, Ir oder Re allein eingesetzt. In einer solchen Kombination beträgt der Anteil des Pt 10-90 %, bevorzugt 15-80 %, besonders bevorzugt 20-70 % des Gesamtgewichts aller Edelmetalle.

Der einzusetzende Katalysator kann weiterhin 1-12 Gew.-%, bevorzugt 2-10 Gew.-%, bezogen auf das Katalysatorgewicht, eines oder mehrerer Alkalimetallhydroxide und/oder Alkalimetallsulfate enthalten, wobei die Hydroxid- und die Sulfatanteile jeweils höchstens 6 Gew.-%, bevorzugt jeweils höchstens 5 Gew.-% betragen. Beispiele für solche Hydroxide und Sulfate sind: Lithiumhydroxid, Natriumhydroxid, Kaliumhydroxid, Rubidiumhydroxid, Caesiumhydroxid, bevorzugt Lithiumhydroxid, Natriumhydroxid, Kaliumhydroxid, besonders bevorzugt Natriumhydroxid oder Kaliumhydroxid; Lithiumsulfat, Natriumsulfat, Kaliumsulfat, Rubidiumsulfat, Caesiumsulfat, bevorzugt Lithiumsulfat, Natriumsulfat, Kaliumsulfat, besonders bevorzugt Natriumsulfat oder Kaliumsulfat.

Die genannten Bestandteile der einzusetzenden Katalysatoren sind auf einem Träger angeordnet. Beispiele für solche Träger sind Aluminiumoxid, Aluminiumspinell, Aktivkohle, Kieselgel, Bentonit, Bimsstein, Zirkonoxid, Titanoxid, Zinkoxid, Magnesiumoxid sowie Oxide der seltenen Erden. Die zuletzt genannten Oxide können auch Dotierungsbestandteile der zuerst genannten Träger sein.

Die genannten Bestandteile der einzusetzenden Katalysatoren sind bevorzugt auf einem Träger aus Aluminiumoxid oder einem Aluminium-Spinell, aufgetragen, besonders bevorzugt auf einem solchen Aluminiumoxid oder Aluminium-Spinell, das mit Mangan und Chrom oder Cer behandelt worden ist. Als Aluminiumoxid kommen insbesondere die α- und die γ- Modifikation infrage. Aluminium-Spinelle sind Verbindungen der Formel

Me(II)Al₂O₄ bzw. Me(I)AlO₂,

in denen Me(II) ein zweiwertiges Metallkation des Eisens, Zinks, Nickels, Kupfers, Kobalts, Cadmiums, Magnesiums oder anderer, bevorzugt des Magnesiums, und Me(I) ein einwertiges Kation, beispielsweise Lithium (Lithium-Aluminium-Spinell), ist. Das Aluminium in den Spinellen kann teilweise durch dreiwertiges Eisen, Chrom oder Mangan ersetzt sein. In bevorzugter Weise wird Al₂O₃, besonders bevorzugt das γ-Al₂O₃, eingesetzt. Ein solcher Träger weist in der besonders bevorzugten Weise einen Gehalt an Chrom und Mangan von zusammen etwa 0,05-8 Gew.-%, bevorzugt 0,2-5 Gew.-%, bezogen auf das Gesamtgewicht des Katalysators, auf. Das Gewichtsverhältnis von Chrom und Mangan beträgt 5:1-1:5, bevorzugt 2:1-1:2. Solche mit Chrom und Mangan behandelten Träger sind aus EP 208 933 bekannt.

Zur Herstellung der beschriebenen Katalysatoren kann in bevorzugter Weise so vorgangen werden, daß auf ein Al₂O₃ in Form von Strangpreßlingen, Pillen oder Kugeln mit Abmessungen von etwa 2-10 mm Verbindungen des Chroms und Mangans aufgebracht werden, der so beaufschlagte Träger auf höhere Temperatur erhitzt wird, anschließend getrennt die dehydrierend wirkenden Metalle und eines oder mehrere der Alkalihydroxide und/oder eines oder mehrere der Alkalisulfate aufgetragen werden; nach jedem Auftragen wird eine Trocknung vorgenommen, im allgemeinen bei 100-140°C bei vermindertem bis normalem Druck, beispielsweise 1-1000 mbar, bevorzugt 10-500 mbar, beispielsweise im Wasserstrahlvakuum.

Das Aufbringen des Chroms und Mangans auf den Katalysatorträger kann beispielsweise durch gemeinsames Ausfällen eines Mangan-Chrom-Hydroxid-Gemisches aus einer Chrom- und Mangansalz-Lösung mit Alkalilauge oder Ammoniak und anschließendes Auswaschen der löslichen Anteile mit Wasser erfolgen. Als Chrom- und Mangansalze kommen insbesondere die Sulfate, Chloride, Acetate und/oder Nitrate der genannten Elemente in Betracht. Die Abscheidung des Chroms und Mangans auf dem Katalysatorträger kann auch als Ammonium-Mangan-Chromat oder Ammonium-Alkali-Mangan-Chromat aus einer Lösung von Hangan(II)-Salzen und Ammoniumbichromat mittels Ammoniak und/oder basischer Alkaliverbindungen erfolgen. Besonders gleichmäßige und festhaftende Abscheidungen erhält man, wenn die Basenzugabe langsam und gleichmäßig unter Vermeidung größerer Konzentrationsunterschiede erfolgt.

Hierzu kann beispielsweise die Fällung mittels Harnstoff unter hydrolisierenden Bedingungen vorgenommen werden, wodurch die Bedingungen der langsamen Basenzugabe besonders gut gewährleistet sind.

Nach dem Aufbringen der Chrom- und Manganverbindungen und der beschriebenen Ausfällung wird der so beaufschlagte Katalysatorträger frei von löslichen Verbindungen gewaschen, bevor er auf höhere Temperaturen (etwa 200-450°C, bevorzugt 250-350°C) erhitzt wird. Nach dieser Temperung ist der mit Chrom und Mangan beaufschlagte Träger bereit zur Tränkung mit den übrigen genannten Katalysatorbestandteilen.

Anstelle des Chroms kann auch Cer oder ein anderes Element aus der Reihe der seltenen Erden verwendet werden. Eine genaue Beschreibung der Herstellung dieses Katalysators findet sich in DE-OS 41 07 395.

Die Tränkung des Katalysatorträgers mit den Metallen beziehungsweise mit Alkalihydroxid und/oder Alkalisulfat (jeweils eines oder mehrere von diesen) erfolgt getrennt. Hierbei kann so vorgangen werden, daß zunächst die Metalle, beispielsweise in Form wäßriger Lösungen ihrer Chloride, Nitrate, Acetate oder anderer geeigneter Salze auf den Träger aufgetränkt werden, wobei nach der Trocknung eine weitere Tränkung mit einer Lösung von Alkalihydroxid und/oder Alkalimetallsulfat erfolgt. Bei dieser Behandlung werden die Metalle in Form ihrer Oxide oder Hydroxide ausgefällt. Das Auftränken des oder der Alkalihydroxide und des oder der Alkalimetallsulfate kann getrennt oder gemeinsam erfolgen. Nach einer abschließenden Trocknung steht der Katalysator zur Verfügung.

Er wird in bevorzugter Weise vor seinem Einsatz durch Behandlung mit Wasserstoff bei erhöhter Temperatur, beispielsweise bei 120-400°C, bevorzugt bei 150-380°C, aktiviert. Diese Aktivierung kann mit besonderem Vorteil in dem Reaktor erfolgen, in welchem anschließend die erfindungsgemäße Herstellung von Diphenylamin erfolgt.

Man kann jedoch auch den Träger zunächst mit einer Alkalihydroxidlösung tränken, anschließend trocknen und auf den so vorbehandelten, basisch eingestellten Katalysatorträger die genannten Salze der Metalle auftragen, wobei im Moment der Tränkung auch die Ausfällung der Metalle in Form ihrer Oxide oder Hydroxide erfolgt. Bei dieser Variante kann das zusätzliche Auftränken von Alkalimetallsulfaten gemeinsam mit dem Alkalihydroxid, vor oder nach der Auftragung des Alkalihydroxids oder als abschließende Tränkung nach dem Auftragen der Metalle erfolgen. Auch hier erfolgt nach jedem Auftranken ein separates Trocknen. Nach der anschließenden Trocknung ist auch hierbei wieder der Katalysator einsatzbereit, kann aber in der beschriebenen Weise vorab auch mit Wasserstoff bei erhöhter Temperatur aktiviert werden.

Anstatt den genannten Träger zur Beaufschlagung mit den genannten Stoffen zu tränken, kann er auch mit geeigneten Lösungen besprüht werden. Die zu all diesen Arbeitsvorgängen erforderlichen Geräte und die Einstellung der gewünschten Beaufschlagung durch Wahl der Menge und Konzentration der Lösungen der genannten Elemente sind dem Fachmann im Prinzip bekannt.

Neben wäßrigen Lösungen kommen grundsätzlich auch alkoholische Lösungen oder Lösungen in niederen Carbonsäuren oder niederen Aminen infrage, sofern die vorgesehenen Salze der Metalle beziehungsweise die basischen Alkalimetallverbindungen oder die Sulfate darin löslich sind.

C₁-C₄-Alkyl oder C₁-C₄-Alkoxy in den Substituenten R¹-R⁴ sind beispielsweise Methyl, Ethyl, Propyl, Isopropyl, Butyl, Isobutyl, Methoxy, Ethoxy, Propoxy, Isopropoxy, Butoxy oder Isobutoxy. In bevorzugter Weise haben die genannten Substituenten 1-2 C-Atome, besonders bevorzugt handelt es sich um Methyl beziehungsweise um Methoxy. In weiterhin bevorzugter Weise bedeutet der Substituent R² beziehungsweise R⁴ Wasserstoff, während die Substituenten R¹ und R³ den genannten Bedeutungsumfang annehmen. In besonders bevorzugter Weise richtet sich das Verfahren auf die Herstellung von nicht substituiertem Diphenylamin.

Die Ausgangsverbindungen Anilin und Cyclohexanon beziehungsweise Anilin und Cyclohexanon/Phenol-Gemisch werden im molaren Verhältnis von 1:4-6:1, vorzugsweise 1:2-5:1 eingesetzt. Die Ausgangsverbindungen werden einzeln oder gemeinsam verdampft und das entstehende Dampfgemisch gegebenenfalls mit Hilfe eines Trägergasstromes an den oben beschriebenen Katalysator gebracht. Trägergase hierfür sind beispielsweise Stickstoff, Wasserstoff, Argon, niedere Kohlenwasserstoffe, wie Methan, Ethan oder Erdgas sowie Gemische hieraus. In bevorzugter Weise werden Stickstoff oder Wasserstoff oder ein Gemisch von ihnen als Trägergas eingesetzt. Das Trägergas wird in einer Menge von 1-100 l/g Ausgangsmaterial, bevorzugt 1-50 l/g Ausgangsmaterial, eingesetzt. Die Katalysatorbelastung wird mit 0,01-1 kg Ausgangsmaterial je 1 Katalysator und Stunde festgesetzt.

Neben den genannten Ausgangsverbindungen Anilin und Cyclohexanon und dem bereits beschriebenen teilweisen Ersatz des Cyclohexanons durch das korrespondierende Phenol können weitere Stoffe, wie N-Cyclohexyliden-anilin oder Dicyclohexylamin oder N-Cyclohexyl-anilin eingesetzt werden.

Das erfindungsgemäße Verfahren wird bei einer Temperatur von 200-450°C, bevorzugt 200-400°C, und einem Druck von 0,1-20 bar, bevorzugt 1-6 bar, in der Gasphase durchgeführt, Die Kombination von Reaktionstemperatur und Reaktionsdruck werden in einer dem Fachmann bekannten Weise so gewählt, daß stets in Gasphase gearbeitet werden kann.

### Beispiel 1

200 g eines handelsüblichen γ-Al₂O₃ (Kugeldurchmesser 2-5 mm) mit einer spez. Oberfläche von 350 m²/g wurde über Nacht mit einer Lösung getränkt, die aus 2,66 g H₂PtCl₆ und 533 g Wasser hergestellt worden war. Der Katalysator wurde dann von der Flüssigkeit abgetrennt und bei 100°C über Nacht getrocknet. Anschließend wurde der Katalysator 3 Stunden bei 350°C im Stickstoffstrom getempert, dann wurde er 3 Stunden im Wasserstoffstrom (50 l/h) bei 350°C reduziert. 100 g des Katalysators wurden 1 Stunde mit einer Lösung getränkt, die aus 5 g Na₂SO₄ und 115 g Wasser hergestellt worden war. Dann wurde die überschüssige Lösung abgetrennt. Nach einer weiteren Zwischentrocknung wurde der Katalysator mit einer Lösung von 3 g NaOH in 35 ml Wasser getränkt und vor dem Einsatz erneut getrocknet.

Ein Reaktionsrohr mit einem Durchmesser von 17 mm und einer Länge von 70 cm wurde in seinem mittleren Teil mit 15 ml (13,6 g) des beschriebenen Katalysators gefüllt; der obere Teil war angefüllt mit Füllkörpern und diente als Verdampfungszone. Bei einer Temperatur von 400°C wurde der Katalysator zunächst 66 Stunden in einem Wasserstoffstrom (20 l/h) aktiviert. Unter Benutzung einer geeichten Dosiervorrichtung wurde bei 340°C ein Gemisch aus 1 Mol Anilin und 2 Mol Cyclohexanon am Katalysator umgesetzt. 486 g der Mischung wurden im Verlauf von 66 Stunden zusammen mit Wasserstoff (2 l/h) und Stickstoff (5 l/h) in das Reaktionsrohr geleitet. Das Reaktionsprodukt wurde kondensiert und gaschromatographisch analysiert. Es hatte folgende Zusammensetzung:

| | |
|---|---|
| Diphenylamin | 46,6 % |
| N-Cyclohexylanilin | 3,7 % |
| N-Cyclohexylidenanilin | 6,7 % |
| Carbazol | 0,8 % |
| Anilin | 18,5 % |
| Phenol | 15,6 % |
| Cyclohexanon | 7,5 % |
| Nebenprodukte | Rest zu 100 % |

### Beispiel 2

γ-Aluminiumoxid (Strangpreßlinge ø=4 mm) mit einer spez. Oberfläche von 230 m²/g wurde in gleicher Weise wie in Beispiel 1 mit H₂PtCl₆, Na₂SO₄ und NaOH imprägniert. 15 ml (9,7 g) des so hergestellten Katalysators wurden unter Benutzung des in Beispiel 1 beschriebenen Reaktionsrohres im Wasserstoffstrom (30 l/h) auf 300°C erwärmt und 18 Stunden bei dieser Temperatur gehalten. Dann wurde die Ofentemperatur des Reaktionsrohres auf 320°C erhöht und die Reaktion durchgeführt. Bei dieser Temperatur wurde ein Gemisch umgesetzt, das Anilin und Cyclohexanon im Molverhältnis 1:1 enthielt. 683 g der Mischung wurden innerhalb von 140 Stunden zusammen mit Wasserstoff (2 l/h) und Stickstoff (5 l/h) in das Reaktionsrohr geleitet. Das Reaktionsprodukt wurde kondensiert und analysiert. Es hatte folgende Zusammensetzung:

| | |
|---|---|
| Diphenylamin | 42,3 % |
| N-Cyclohexylanilin | 47,8 % |
| N-Cyclohexylidenanilin | 6,6 % |
| Anilin | 1,9 % |
| Phenol | 0,1 % |
| Nebenprodukte | Rest zu 100 % |

Anschließend wurde die Temperatur des Reaktionsofens auf 370°C erhöht, und im Verlauf von 164 Stunden wurden weitere 901 g des Anilin-Cyclohexanon-Gemisches über den Katalysator geleitet. Das kondensierte Reaktionsprodukt hatte folgende Zusammensetzung:

| | |
|---|---|
| Diphenylamin | 57,1 % |
| N-Cyclohexylanilin | 28,4 % |
| N-Cyclohexylidenanilin | 10,7 % |
| Anilin | 1,0 % |
| Phenol | 0,5 % |
| Nebenprodukte | Rest |

### Beispiel 3

400 g eines handelsüblichen γ-Al₂O₃ (Kugeldurchesser 3-8 mm) mit einer spez. Oberfläche von 330 m²/g wurden mit einer Lösung getränkt, die aus 10 g H₂PtCl₆ und 1.060 g H₂O hergestellt wurde. Es wurde dabei so verfahren wie in Beispiel 1.

100 g des reduzierten Pt-Katalysators wurden aber diesmal mit einer Lösung getränkt, die aus 2 g Re₂O₇ und 32 g Wasser hergestellt worden war, Nach einer weiteren Zwischentrocknung wurden 20 ml (13 g) des so nachbehandelten Pt-Katalysators im Wasserstoffstrom (60 l/h) 66 Stunden bei 400°C aktiviert. Dann wurde ein Gemisch, das Anilin und Cyclohexanon im Molverhältnis 1:2 enthielt, zur Diphenylherstellung gemäß Beispiel 1 umgesetzt. Dabei wurden im Verlauf von 71 Std. 472 g des Eduktes zusammen mit Wasserstoff (2 l/h) und Stickstoff (5 l/h) in das Reaktionsrohr geleitet. Das kondensierte Reaktionsprodukt hatte folgende Zusammensetzung:

| | |
|---|---|
| Diphenylamin | 36,6 % |
| N-Cyclohexylanilin | 3,8 % |
| N-Cyclohexylidenanilin | 20,5 % |
| Cyclohexanon | 9,7 % |
| Anilin | 14,5 % |
| Phenol | 8,9 % |
| Nebenprodukte | Rest |

### Beispiel 4

100 g (63 ml) Zirkondioxid-Tabletten der Größe 1/8" und einer spez. Oberfläche von 50 m²/g wurden mit einer Lösung getränkt, die aus 2,08 g Pd-Acetat und 25 g Methylenchlorid hergestellt wurde. Von den bei 120°C getrockneten Tabletten wurden 20 ml (33,5 g) zur Umsetzung von Anilin und Cyclohexanon im Molverhältnis 1,7:1 verwendet. Bei 270°C wurden im Verlauf von 195 Stunden 701 g von dem Anilin-Cyclohexanon-Gemisch zur Reaktion gebracht, Das kondensierte Reaktionsprodukt hatte folgende Zusammensetzung:

| | |
|---|---|
| Diphenylamin | 27,1 % |
| N-Cyclohexylanilin | 10,6 % |
| N-Cyclohexylidenanilin | 0,1 % |
| Phenol | 36,2 % |
| Anilin | 11,9 % |
| Nebenprodukte | Rest |

### Beispiel 5

100 g eines handelsüblichen γ-Al₂O₃ mit einer spez. Oberfläche von 150 m²/g, das in Form eines Extrudats (1/16") vorlag, wurden in gleicher Weise wie das Aluminiumoxid in Beispiel 1 zunächst mit Pt, dann mit Na₂SO₄ und NaOH imprägniert.

Mit 50 ml (32 g) des hier hergestellten Katalysators wurden Anilin und Cyclohexanon im Molverhältnis 1:1 umgesetzt. 489 g der Mischung wurden im Verlauf von 44 Stunden gleichmäßig durch den auf 400°C erhitzten Katalysator geleitet. Das kondensierte Reaktionsprodukt hatte folgende Zusammensetzung:

| | |
|---|---|
| Diphenylamin | 37,9 % |
| N-Cyclohexylanilin | 0,03 % |
| N-Cyclohexylidenanilin | 0,1 % |
| Phenol | 25,8 % |
| Anilin | 30,9 % |
| Nebenprodukte | Rest % |

### Beispiel 6

100 ml eines mit Mangan und Chrom gemäß EP-Anmeldung 0 208 933, Beispiel 1, beaufschlagten γ-Al₂O₃ in Kugelform (2 bis 6 mm) wurden mit 0,8 g Pt in Form einer H₂PLCl₆-Lösung imprägniert. 20 g (17 g) des getrockneten Katalysators wurden in einem Wasserstoffstrom (30 l H₂/h) 70 Stunden bei einer Ofentemperatur von 340°C aktiviert. Bei dieser Temperatur wurden Anilin und Cyclohexanon im Molverhältnis 1:1 umgesetzt. 30 g der Mischung wurden innerhalb von 26,5 Stunden zusammen mit Stickstoff (5 l/h) durch das Reaktionsrohr geleitet. Das Reaktionsprodukt wurde kondensiert und analysiert. Er hatte folgende Zusammensetzung:

| | |
|---|---|
| Diphenylamin | 59,1 % |
| N-Cyclohexylanilin | 1,3 % |
| N-Cyclohexylidenanilin | 0,4 % |
| Carbazol | 2,7 % |
| Phenol | 13,2 % |
| Anilin | 21,0 % |
| Cyclohexanon | 0,8 % |
| Nebenprodukte | Rest % |

### Beispiel 7

400 g eines handelsüblichen γ-Al₂O₃ mit einer spezifischen Oberfläche von 350 m²/g und einem Kugeldurchmesser von 2 bis 6 mm wurden mit einer Lösung getränkt, die aus 24,8 g Ce(NO₃)₃ x 6 H₂O, 35,7 g Mn(CH₃COO)₂ x 4 H₂O und 100 g Wasser hergestellt worden war. Das getränkte Al₂O₃ wurde getrocknet und anschließend 3 Stunden bei 400°C getempert.

200 g des so hergestellten Katalysatorträgers wurden mit einer Lösung getränkt, die aus 8,3 g Pd(CH₃COO)₂ und 62 g Methylenchlorid hergestellt worden war. Nach einer Zwischentrocknung wurde der Katalysator nochmals mit einer Lösung getränkt, die aus 6 g K₂SO₄ und 60 g Wasser hergestellt worden war. Nach einer erneuten Trocknung bei 120°C wurden 200 ml (168 g) des Pd-Katalysators in ein senkrecht angeordnetes Glasrohr (ø = 32 mm) zur Umsetzung von Anilin und Cyclohexanon im Molverhältnis 1:1 eingesetzt. Im Verlauf von 24 Stunden wurden 808 g des Gemisches über den 330-340°C heißen Katalysator geleitet. Das kondensierte Reaktionsprodukt hatte folgende Zusammensetzung:

| | |
|---|---|
| Diphenylamin | 64,3 % |
| N-Cyclohexylanilin | 0,1 % |
| N-Cyclohexylidenanilin | 0,03 % |
| Carbazol | 0,9 % |
| Phenol | 11,2 % |
| Anilin | 20,9 % |
| Nebenprodukte | Rest % |

## Patentansprüche

1. Verfahren zur Herstellung von Diphenylaminen der Formel 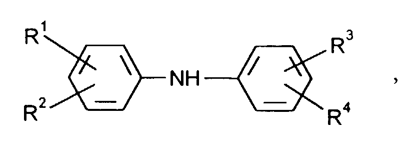 in der
R¹, R², R³ und R⁴ unabhängig voneinander Wasserstoff, C₁-C₄-Alkyl oder C₁-C₄-Alkoxy bedeuten,
dadurch gekennzeichnet, daß Aniline der Formel 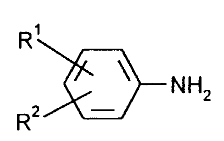 mit Cyclohexanonen der Formel 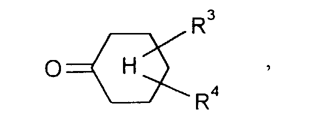 die sich im Gemisch mit recyclisierten Reaktionsprodukten befinden können, wobei in den Formeln die Reste R¹ bis R⁴ die obige Bedeutung haben, und wobei Anilin:Cyclohexanon im molaren Verhältnis von 1:4-6:1, bevorzugt 1:2-5:1, vorliegen, an einem 0,05-5 Gew.-%, bevorzugt 0,05-4 Gew.-%, besonders bevorzugt 0,1-3 Gew.-%, bezogen auf das Gesamtgewicht des Katalysators, eines oder mehrere dehydrierend wirkende(s) Metall(e) enthaltenden Trägerkatalysator bei 200-450°C und 0,1-20 bar in der Gasphase umgesetzt werden, wobei das (die) Metall(e) der Gruppe aus Ru, Pd, Os, Ir, Pt, Fe, Co, Ni, Re, Mn, Ce, Cu, Ag und Cr angehören.

2. Verfahren nach Anspruch 1, dadurch gekennzeichnet, daß die Umsetzung bei 200-400°C durchgeführt wird.

3. Verfahren nach Anspruch 1, dadurch gekennzeichnet, daß die Umsetzung bei 0,1-6 bar durchgeführt wird.

4. Verfahren nach Anspruch 1, dadurch gekennzeichnet, daß das (die) Metall(e) der Gruppe aus Pd, Ir, Pt, Co, Ni, Re, Mn, Ce und Cr angehören.

5. Verfahren nach Anspruch 4, dadurch gekennzeichnet, daß Pt in Kombination mit Pd, Ir oder Re eingesetzt wird.

6. Verfahren nach Anspruch 1, dadurch gekennzeichnet, daß der Träger Aluminiumoxid, bevorzugt ein mit Mangan und Chrom oder Cer behandeltes Aluminiumoxid ist.

7. Verfahren nach Anspruch 1, dadurch gekennzeichnet, daß der Katalysator zusätzlich 1-12 Gew.-%, bezogen auf das Katalysatorgesamtgewicht, eines oder mehrere Alkalimetallhydroxide und/oder Alkalimetallsulfate enthält, wobei die Hydroxide und Sulfate jeweils höchstens 6 Gew.-% betragen.

8. Verfahren nach Anspruch 1, dadurch gekennzeichnet, daß das Cyclohexanon zu 0-20 mol-% durch das korrespondierende Phenol ersetzt wird.

## Claims

1. Process for preparing diphenylamines of the formula 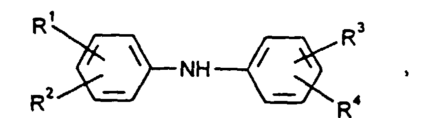 in which
R¹, R², R³ and R⁴ denote, independently of one another, hydrogen, C₁-C₄-alkyl or C₁-C₄-alkoxy,
characterised in that anilines of the formula 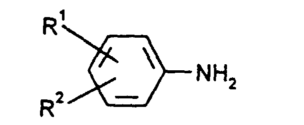 are reacted with cyclohexanones of the formula 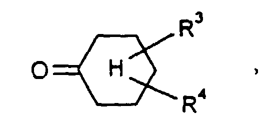 which may be present mixed with recycled reaction products, in the formulae the radicals R¹ to R⁴ having the above meaning and aniline and cyclohexanone being present in a molar ratio of 1:4-6:1, preferably 1:2-5:1, over a supported catalyst containing 0.05-5 % by weight preferably 0.05-4 % by weight, particularly preferably 0.1-3 % by weight, based on the total weight of the catalyst, of one or more metals having a dehydrogenating action, at 200-450°C and at 0.1-20 bar in the gas phase, the metal(s) belonging to the group comprising Ru, Pd, Os, Ir, Pt, Fe, Co, Ni, Re, Mn, Ce, Cu, Af and Cr.

2. Process according to Claim 1, characterised in that the reaction is carried out at 200-400°C.

3. Process according to Claim 1, characterised in that the reaction is carried out at 0.1-6 bar.

4. Process according to Claim 1, characterised in that the metal(s) belong(s) to the group comprising Pd, Ir, Pt, Co, Ni, Re, Mn, Ce and Cr.

5. Process according to Claim 4, characterised in that Pt is used in combination with Pd, Ir or Re.

6. Process according to Claim 1, characterised in that the support is aluminium oxide, preferably an aluminium oxide treated with manganese and chromium or cerium.

7. Process according to Claim 1, characterised in that the catalyst additionally contains 1-12 % by weight, based on the total weight of the catalyst, oif one or more alkali metal hydroxides and/or alkali metal sulphates, the hydroxides and sulphates in each case being present in an amount of at most 6 % by weight.

8. Process according to Claim 1, characterised in that the cyclohexanone is replaced in an amount of 0-20 mol/% by the corresponding phenol.

## Revendications

1. Procédé de préparation des diphénylamines de formule 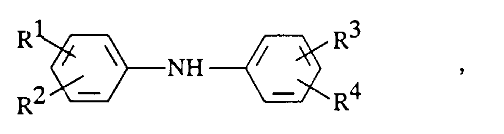 dans laquelle
R¹, R², R³ et R⁴ représentent chacun, indépendamment les uns des autres, l'hydrogène, un groupe alkyle en C₁-C₄ ou alcoxy en C₁-C₄,
caractérisé en ce que l'on fait réagir en phase gazeuse des anilines de formule 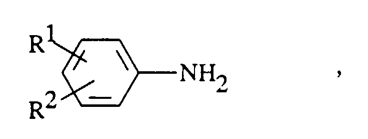 avec des cyclohexanones de formule 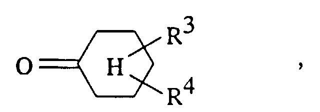 éventuellement en mélange avec les produits de réaction recyclés, les symboles R¹ à R⁴ ayant les significations indiquées ci-dessus, et l'aniline et la cyclohexanone étant présentes à un rapport molaire de 1:4 à 6:1, de préférence de 1:2 à 5:1, sur un catalyseur sur support contenant 0,05 à 5 %, de préférence 0,05 à 4 % et plus spécialement 0,1 à 3 % de son poids d'un ou plusieurs métaux à effet déshydrogénant, à des températures de 200 à 450°C et des pressions de 0,1 à 20 bar, les métaux en question appartenant au groupe formé par Ru, Pd, Os, Ir, Pt, Fe, Co, Ni, Re, Mn, Ce, Cu, Ag et Cr.

2. Procédé selon la revendication 1, caractérisé en ce que la réaction est réalisée à des températures de 200 à 400°C.

3. Procédé selon la revendication 1, caractérisé en ce que la réaction est réalisée sous une pression de 0,1 à 6 bar.

4. Procédé selon la revendication 1, caractérisé en ce que les métaux appartiennent au groupe formé par Pd, Ir, Pt, Co, Ni, Re, Mn, Ce et Cr.

5. Procédé selon la revendication 4, caractérisé en ce que l'on utilise Pt en combinaison avec Pd, Ir ou Re.

6. Procédé selon la revendication 1, caractérisé en ce que le support consiste en une alumine, de préférence une alumine traitée par du manganèse et du chrome et du cérium.

7. Procédé selon la revendication 1, caractérisé en ce que le catalyseur contient en outre de 1 à 12 % de son poids total d'un ou plusieurs hydroxydes de métaux alcalins et/ou sulfates de métaux alcalins, les hydroxydes et sulfates étant présents en proportion maximale de 6 % en poids pour chacune des deux classes de composés.

8. Procédé selon la revendication 1, caractérisé en ce que la cyclohexanone est remplacée en proportion de 0 à 20 mol % par le phénol correspondant.
